# EUROPEAN PATENT APPLICATION

(11) **EP 3 431 402 A1**
(43) Date of publication of application: **23.01.2019**
(21) Application number: 17766207.9
(22) Date of filing: 16.02.2017
(51) Int. Cl.: B65B 55/04, A61L 2/08, B65B 55/08, G21K 5/00, G21K 5/04, G21K 5/10

(54) **ELECTRON BEAM STERILIZATION APPARATUS**

(30) Priority: 18.03.2016 JP 2016054586
(71) Applicant: Hitachi Zosen Corporation, Osaka-shi, Osaka 559-8559 (JP)
(72) Inventor: SASAKI, Kazuaki, Osaka-shi, Osaka 559-8559 (JP); YOKOO, Kazuyuki, Osaka-shi, Osaka 559-8559 (JP)
(74) Representative: Engelhardt, Harald
(86) International application number: PCT/JP2017/005675
(87) International publication number: WO 2017/159200

(57) **Abstract**

A container outer-surface sterilizer is provided for sterilization with an electron beam emitted from an electron beam irradiation device to a preform (P). The container outer-surface sterilizer includes a container conveying device (21) that conveys the held preform (P), a container rotating device (26) that is provided on the container conveying device (21) and rotates the preform (P), and a magnetic-type driving force transmission mechanism (31) that transmits a driving force in a noncontact manner between a strip magnet assembly (32) and a ring magnet assembly (33) of the container rotating device (26). The magnetic-type driving force transmission mechanism (31) includes a magnet array provided for each of the strip magnet assembly (32) and the ring magnet assembly (33) that are moved while being opposed to each other with a predetermined gap, the magnet array including magnets alternately arranged with different magnetic poles.

## Description

### Technical Field

The present invention relates to an electron beam sterilization apparatus that sterilizes an irradiation object, e.g., a container (also meaning a subject and a preform) by the irradiation of an electron beam.

### Background Art

In the case of sterilization on the outer surface of an irradiation object, the irradiation object is held by a holding part of a container conveying device and then the irradiation object is rotated while being conveyed in the irradiated region of an electron beam, thereby sterilizing the overall outer surface of the object. Such a technique is proposed in, for example, Patent Literatures 1 and 2.

In the conveying device, a plurality of irradiation objects are sequentially held by the holding part and then the irradiation objects are rotated while being conveyed. However, the provision of a rotating device for each of the irradiation objects may increase the size of the container conveying device. Thus, the irradiation objects are each rotated via a contact-type driving force transmission mechanism including gears and cams or a transmission shaft between the rotating device and the holding part.

### Citation List

### Patent Literature

Patent Literature 1: Japanese Patent Laid-Open No. 2014-129142
Patent Literature 2: Japanese Patent Laid-Open No. 2007-297067

### Summary of Invention

### Technical Problem

In the case of a sterilization apparatus for containers and preforms, the sterilization apparatus and a filling device or a blow molding machine constitute a series of apparatuses. In such an apparatus, typically, measures for preventing the occurrence of dust or dirt in the atmosphere of sterilization are implemented. However, using a contact-type transmission mechanism may cause dust or dirt by abrasion, disadvantageously increasing the probability of contamination on containers and preforms.

The present invention is devised to solve the problem. An object of the present invention is to provide an electron beam sterilization apparatus that is free from dust or dirt and thus does not have an adverse effect, e.g., contamination on an irradiation object.

### Solution to Problem

The present invention is an electron beam sterilization apparatus for sterilization with an electron beam emitted from an electron beam irradiation device to an irradiation object,
the sterilization apparatus including:
a conveying device that conveys a held irradiation object;
a rotating device that is provided on the conveying device and rotates the irradiation object; and
a magnetic-type driving force transmission mechanism that transmits a driving force in a noncontact manner between a driving member and a receiving movement member of the rotating device,
the driving force transmission mechanism including a magnet array provided for each of the driving member and the receiving movement member that are moved while being opposed to each other with a predetermined gap, the magnet array including magnets alternately arranged with different magnetic poles.

With this configuration, in the rotating device, the irradiation object is rotated via the magnetic-type driving force transmission mechanism and a driving force is transmitted in a noncontact manner by the magnetic-type driving force transmission mechanism during sterilization. This can eliminate the need for a contact part or a friction part for transmitting a driving force, thereby preventing the occurrence of dust or dirt caused by contact or abrasion. Thus, the irradiation object is not contaminated by dust or dirt.

In this case, the "driving force transmission mechanism" includes a structure for transmitting power between members by using a magnetic force and a driving force conversion mechanism for changing a direction of motion, e.g., from a linear motion to a rotational motion between members.

This configuration preferably includes an interference preventing member between the magnetic-type driving force transmission mechanism and the irradiation object, the interference preventing member preventing a magnetic force generated in the driving force transmission mechanism from adversely affecting the electron beam.

This configuration can prevent a magnetic force generated from the magnetic-type driving force transmission mechanism from adversely affecting an electron beam, achieving stable sterilization with an electron beam.

The interference preventing member of this configuration preferably has a magnetic-force reduction distance for reducing a magnetic force, between the magnetic-type driving force transmission mechanism and the irradiation object. The magnetic-force reduction distance is secured to reduce a magnetic force, achieving stable sterilization.

The interference preventing member of this configuration is preferably a magnetic field shield disposed between the magnetic-type driving force transmission mechanism and the irradiation object, the magnetic field shield shielding a magnetic field generated from the magnetic-type driving force transmission mechanism. With this configuration, a magnetic force generated from the magnetic-type driving force transmission mechanism can be shielded by the magnetic field shield or can be reduced, achieving stable sterilization with an electron beam.

In this configuration, the magnet array is preferably covered with a coating member made of a corrosion-resisting material having low magnetic permeability.

With this configuration, the coating member can prevent corrosion of the magnet array in an atmosphere of corrosive gas such as ozone gas generated by collision of an electron beam with oxygen in the air and nitric acid gas generated by electron beam energy that causes a reaction of nitrogen, oxygen, and hydrogen in the air.

In this configuration, the driving member is preferably a strip magnet assembly disposed on a fixed-side member along the conveying direction of the irradiation object, and the receiving movement member is preferably a ring magnet assembly provided on a movable-side member of the conveying device.

With this configuration, the ring magnet assembly is moved along the strip magnet assembly, thereby converting a linear motion into a rotational motion in a noncontact manner by using the magnetic force of the magnet assembly. This can rotate the ring magnet assembly.

This configuration preferably further includes a chamber surrounding the electron beam irradiation device and the irradiation object with a partition wall that is capable of shielding an X-ray with low magnetic permeability and is made of a corrosion-resisting material. Moreover, the magnet array of the receiving movement member is preferably disposed in the chamber and the magnet array of the driving member is preferably disposed outside the chamber via the coating member made of a corrosion-resisting material having low magnetic permeability.

With this configuration, the driving member having the magnet array is combined with the partition wall of the chamber or is disposed outside the partition wall, thereby preventing corrosive gas from causing corrosion without reducing a magnetic force.

### Advantageous Effect of Invention

According to the present invention, the power transmission mechanism is driven in a noncontact manner, thereby preventing the occurrence of dust or dirt. Thus, the irradiation object is not contaminated.

### Brief Description of Drawings

[FIG. 1] FIG. 1 is a schematic plan view showing a first embodiment of a container outer-surface sterilizer in an electron beam sterilization apparatus according to the present invention.
[FIG. 2A] FIG. 2A is a cross-sectional view showing a conveying device of the container outer-surface sterilizer in an outer-surface irradiation region.
[FIG. 2B] FIG. 2B is an image of the conveying device of the container outer-surface sterilizer in plan view and a ring magnet assembly captured by an imaging device.
[FIG. 3] FIG. 3 is a front cross-sectional view showing the conveying devices in the outer-surface irradiation region.
[FIG. 4] FIG. 4 is a cross-sectional view showing a strip magnet assembly and a ring magnet assembly in the magnetic-type driving force transmission mechanism in plan view.
[FIG. 5] FIG. 5 is a schematic plan view showing a second embodiment of a container inner-surface sterilizer in an electron beam sterilization apparatus according to the present invention.
[FIG. 6] FIG. 6 is a side view showing the principal part of a container rotating device in an inner-surface irradiation region.
[FIG. 7] FIG. 7 is a partial cross-sectional view showing the principal part of the container rotating device in the inner-surface irradiation region in plan view.
[FIG. 8] FIG. 8 is a schematic cross-sectional view showing the principal part of a container lifting device in the inner-surface irradiation region.
[FIG. 9A] FIG. 9A is a cross-sectional view showing a modification of the magnetic-type driving force transmission mechanism and a magnet assembly according to the first modification.
[FIG. 9B] FIG. 9B is a cross-sectional view showing a modification of the magnetic-type driving force transmission mechanism and a magnet assembly according to the second modification.
[FIG. 10A] FIG. 10A is a schematic cross-sectional view showing a third modification of the magnetic-type driving force transmission mechanism in the inner-surface irradiation region.
[FIG. 10B] FIG. 10B is an enlarged side view showing a third modification of the magnetic-type driving force transmission mechanism and a strip magnet assembly and a ring magnet assembly.
[FIG. 10C] FIG. 10C is a plan view showing the third modification of the magnetic-type driving force transmission mechanism and a container rotating device.
[FIG. 11A] FIG. 11A is a partial cross-sectional view showing a third embodiment of a container outer-surface sterilizer in an electron beam sterilization apparatus and a magnetic-type driving force transmission mechanism in plan view according to the present invention.
[FIG. 11B] FIG. 11B is a side view showing the third embodiment of the container outer-surface sterilizer in the electron beam sterilization apparatus according to the present invention.
[FIG. 12] FIG. 12 is a schematic plan view showing the third embodiment of the container outer-surface sterilizer.

### Description of Embodiments

In an electron beam sterilization apparatus according to the present invention, irradiation objects to be sterilized include subjects such as laboratory ware, preforms before container molding, and containers before aseptic filling of contents. These irradiation objects are conveyed or moved up and down while being rotated, so that at least one of the outer surface and the inner surface of the object is sterilized by irradiation with an electron beam. Moreover, a driving force transmission mechanism for rotating the irradiation objects is a magnetic type that can be driven in a noncontact manner. Thus, a driving force is transmitted or converted in a noncontact manner so as to prevent the occurrence of dust or dirt caused by contact or abrasion.

Furthermore, an interference preventing member is provided to prevent a magnetic force generated in the magnetic-type driving force transmission mechanism from adversely affecting an electron beam.

An electron beam emitted into the air initiates a chemical reaction in the air, thereby generating corrosive gas such as ozone gas and nitric acid gas. Thus, it is known that a conveying device and the inside of a chamber covering, for example, an irradiation object are placed in an atmosphere of corrosive gas. For this reason, the magnetic-type driving force transmission mechanism has an anti-corrosive structure that is not subjected to corrosion in an atmosphere of corrosive gas.

### First Embodiment

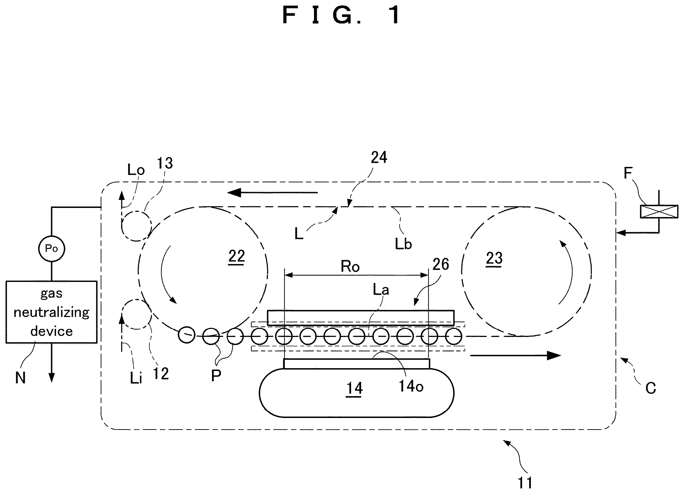

Referring to FIGS. 1 to 4, a first embodiment of a container outer-surface sterilizer according to the present invention will be described below.

As shown in FIG. 1, the electron beam sterilization apparatus is a container outer-surface sterilizer 11 for externally sterilizing preforms P before containers are formed by blow molding. The preforms P are irradiation objects. The container outer-surface sterilizer 11 is covered with a chamber C for shielding corrosive gas, the chamber C surrounding the atmosphere of corrosive gas. Corrosive gas in the chamber C is released into the atmosphere through a gas neutralizing device N for removing toxic substances from gas fed by an exhaust pump Po. Additional air is introduced into the chamber C through a filter F for removing impurities.

### [Container conveying device]

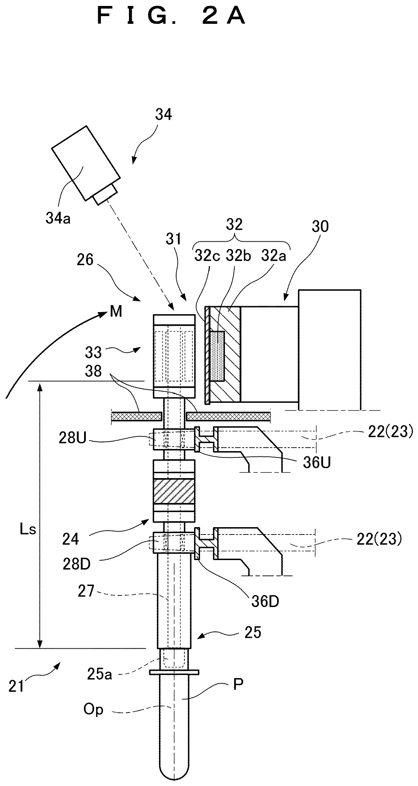

As shown in FIG. 2A, a container conveying device (conveying device) 21 for conveying the preforms P includes a first wheel (rotating body) 22 and a second wheel (rotating body) 23 in a pair, an endless rotating member (movable member) 24 wound between the first wheel 22 and the second wheel 23, and container holding parts 25 provided at regular intervals on the rotating member 24. The preform P fed from a feed passage Li is sent to a linear first half part La of a conveying passage L while being held by the container holding part 25 on a feed wheel 12. The preform P is then sterilized on the linear first half part La by irradiation with an electron beam from an electron beam irradiation device 14. Subsequently, the preform P is sent from the second wheel 23 to a linear second half part Lb and then is sent from the first wheel 22 to a discharge passage Lo.

On the linear first half part La, an outer-surface irradiation region (electron beam irradiation region) Ro is set to be irradiated with an electron beam from the electron beam irradiation device 14. The electron beam irradiation device 14 is installed for the outer-surface irradiation region Ro. The preform P conveyed along the linear first half part La is then irradiated with an electron beam from an irradiation opening 14o of the electron beam irradiation device 14. In the outer-surface irradiation region Ro, the preform P is rotated about a container axis Op by the container rotating device (rotating device) 26 provided in the container conveying device 21, thereby evenly sterilizing the outer peripheral surface of the preform P.

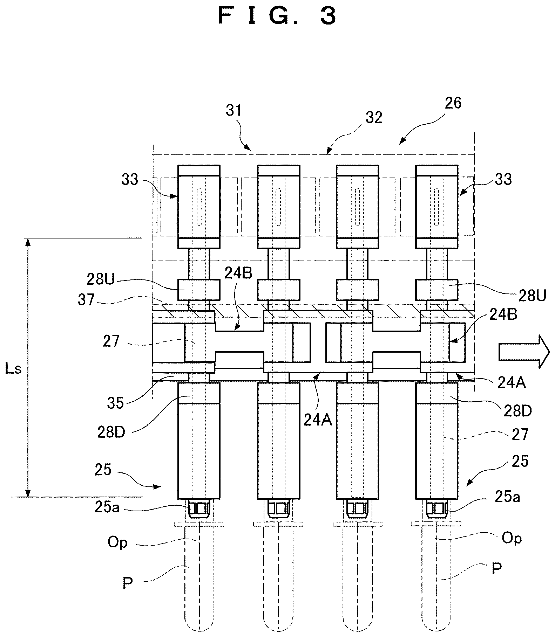

As shown in FIGS. 2A and 3, the rotating member 24 of the container conveying device 21 includes two kinds of chain members 24A and 24B that have different H shapes in side view and are connected so as to bend in a horizontal plane via a spindle 27 perpendicular to the conveying direction. The spindle 27 includes the container holding part 25 provided at the lower end of the spindle 27, wheel engaging rings 28U and 28D rotatably mounted on and under the chain members 24A and 24B via bearings, and the container rotating device 26 provided on the upper end of the spindle 27. The wheel engaging rings 28U and 28D are vertically arranged because the first wheel 22 and the second wheel 23 each include a pair of sprockets vertically spaced at a predetermined interval.

The container holding part 25 includes a flexible retainer 25a that is an elastic body detachably attached to the mouth of the preform P. The preform P is lifted by a lifting mechanism (not shown) provided on the first wheel 22, so that the retainer 25a is fit into the mouth of the preform P. Alternatively, the preform P is moved down to remove the retainer 25a from the mouth of the preform P.

### [Magnetic-type driving force transmission mechanism]

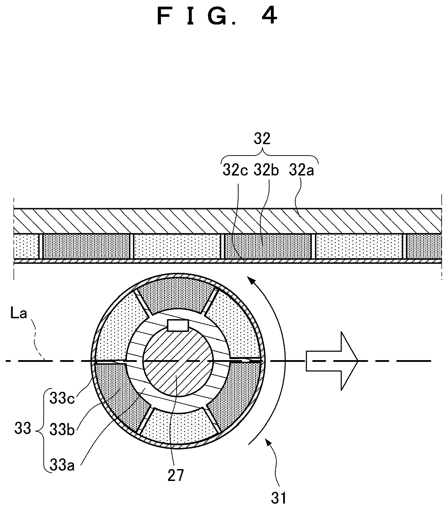

The container rotating device 26 includes a magnetic-type driving force transmission mechanism 31 that converts a linear motion of the rotating member 24, which is moved on the linear first half part La by the container conveying device 21, into a rotational motion of the container holding part 25 in a noncontact manner. As shown in FIG. 4, the magnetic-type driving force transmission mechanism 31 includes a linear strip magnet assembly 32 that is attached along the conveying direction to a support frame 30 serving as a fixed-side member of the container conveying device 21 and a driving member. A ring magnet assembly 33 is fixed to the spindle 27 serving as a movable-side member of the container conveying device 21 and a receiving movement member. In the outer-surface irradiation region Ro, the ring magnet assembly 33 moves with a predetermined gap (a gap between the strip magnet assembly 32 and the ring magnet assembly 33) along the strip magnet assembly 32, thereby converting a linear motion of the rotating member 24 into a rotational motion of the spindle 27 by using a magnetic force.

As shown in FIG. 4, the strip magnet assembly 32 includes a magnet mounting member 32a disposed on the support frame 30 along the conveying direction, a linear strip magnet array 32b that are S-pole and N-pole magnets alternately arranged at regular intervals on the magnet mounting member 32a in the conveying direction, and a cover member 32c that is made of corrosion-resisting materials having low magnetic permeability and covers the strip magnet array 32b. The ring magnet assembly 33 includes a magnet support cylinder 33a fixed to the spindle 27 via a key, a ring magnet array 33b that includes S-pole and N-pole magnets alternately arranged at regular intervals (angles) on the outer periphery of the magnet support cylinder 33a, and a coating member 33c that is made of corrosion-resisting materials having low magnetic permeability and covers the ring magnet array 33b. The cover member 32c and the coating member 33c made of stainless steel or a titanium alloy may be coating layers coated with a stainless material or a titanium alloy. The cover member 32c and the coating member 33c prevent the strip magnet array 32b and the ring magnet array 33b from being corroded by corrosive gas, e.g., nitric acid gas containing liquid nitric acid or ozone gas.

With this configuration, when the rotating member 24 is linearly moved in the linear first half part La in the conveying direction by the container conveying device 21, the magnetic forces of the strip magnet array 32b and the ring magnet array 33b are applied to rotate the spindle 27, so that the preform P held by the retainer 25a of the container holding part 25 is rotated about the container axis Op. The rotating preform P is irradiated with an electron beam by the electron beam irradiation device 14. Thus, the electron beam is evenly emitted to the outer peripheral surface of the preform P.

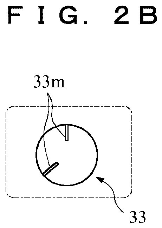

In the event of an insufficient rotation of the preform P, a part of the preform P may be insufficiently irradiated with an electron beam. The insufficiently sterilized preform P may contaminate a blow molding machine located downstream. Thus, as shown in FIGS. 2A and 2B, a container rotation monitoring device 34 is provided to monitor the rotation of the preform P through the spindle 27. The rotation monitoring device 34 captures an image of, for example, marks 33m on the top of the ring magnet assembly 33 by means of an imaging device 34a at high speed and compares the captured image and an image of a normal rotation, thereby monitoring whether or not the preform P has been rotated by a predetermined amount. As a matter of course, the rotation of the spindle 27 is not always monitored using a visual image. A known noncontact detector may be used with ultrasonic waves or radar and the like.

In the outer-surface irradiation region Ro of the linear first half part La, the magnets are used for the magnetic-type driving force transmission mechanism 31 and thus the ring magnet assembly 33 is always attracted to the strip magnet assembly 32, thereby generating a moment M clockwise on the rotating member 24 as shown in FIG. 2A. Typically, on the conveying passage L, the orientation of the container holding part 25 is kept by a guide roller or a magnetic-type orientation retaining mechanism, which is not shown. In this state, however, the configuration cannot deal with the moment M. In order to suppress the inclination of the rotating member 24, inclination regulating rails 36U and 36D are installed over the outer-surface irradiation region Ro so as to regulate inward displacements (to the support frame 30) of the wheel engaging rings 28U and 28D vertically arranged on the rotating member 24. Furthermore, outside the conveying passage L (opposite from the support frame 30), a floating prevention rail 37 in contact with the top surface of an external guide roller, which is not shown, is installed over the outer-surface irradiation region Ro so as to regulate floating of the external guide roller. In this way, in the outer-surface irradiation region Ro, the inclination regulating rails 36U and 36D and the floating prevention rail 37 can prevent the spindles 27 from inclining toward the inside of the conveying passage L.

Moreover, a magnetic force generated from the magnetic-type driving force transmission mechanism 31 may adversely affect the sterilization of the preform P. For example, the radiation direction of an electron beam from the electron beam irradiation device 14 may be deflected so as to cause an uneven irradiation amount on the preform P. As a solution to prevent interference, a magnetic force reduction distance Ls for reducing the magnetic force is secured from the magnetic-type driving force transmission mechanism 31 to the preform P, thereby preventing the magnetic force of the magnetic-type driving force transmission mechanism 31 from affecting the irradiation of the preform P with an electron beam.

As another solution to prevent interference, as shown in FIG. 2A, a magnetic field shield 38 made of high magnetic permeability materials, i.e., Mu-metal or permalloy that shield a magnetic force is mounted in a space between the magnetic-type driving force transmission mechanism 31 and the irradiation opening 14o and the preform P concurrently with or instead of the securing of the magnetic force reduction distance Ls. The surface of the magnetic field shield 38 may be covered with surface materials including an anti-corrosive material. As a matter of course, the magnetic field shield 38 may be mounted when the magnetic force reduction distance Ls is secured.

### [Effects of First Embodiment]

According to the first embodiment, the preform P held by the container holding part 25 is rotated about the container axis Op and is sterilized by irradiation with an electron beam. At this point, the container rotating device 26 rotates the preform P with a driving force transmitted in a noncontact manner by the magnetic-type driving force transmission mechanism 31, thereby preventing the occurrence of dust or dirt caused by contact or abrasion during power transmission. Thus, the preform P is not contaminated by dust or dirt.

In the container rotating device 26, by means of the strip magnet assembly 32 linearly provided on the support frame 30 of the container conveying device 21 and the magnetic-type driving force transmission mechanism 31 including the ring magnet assembly 33 attached to the spindle 27 of the rotating member 24, a linear motion of the rotating member 24 along the linear strip magnet assembly 32 is converted into a rotational motion of the ring magnet assembly 33 in a noncontact manner, thereby preventing the occurrence of dust or dirt caused by contact or abrasion.

As a solution to prevent interference, at least one of the magnetic field shield 38 and the magnetic force reduction distance Ls is set between the magnetic-type driving force transmission mechanism 31 and the preform P, thereby reducing a magnetic field generated from the magnets of the magnetic-type driving force transmission mechanism 31. This can stably radiate an electron beam so as to achieve even sterilization.

Furthermore, the strip magnet array 32b and the ring magnet array 33b are covered with the cover member 32c that is made of corrosion-resisting materials having low magnetic permeability, the coating member 33c, or a coating, thereby preventing corrosion in an atmosphere of corrosive gas such as ozone gas and nitric acid gas.

### Second Embodiment

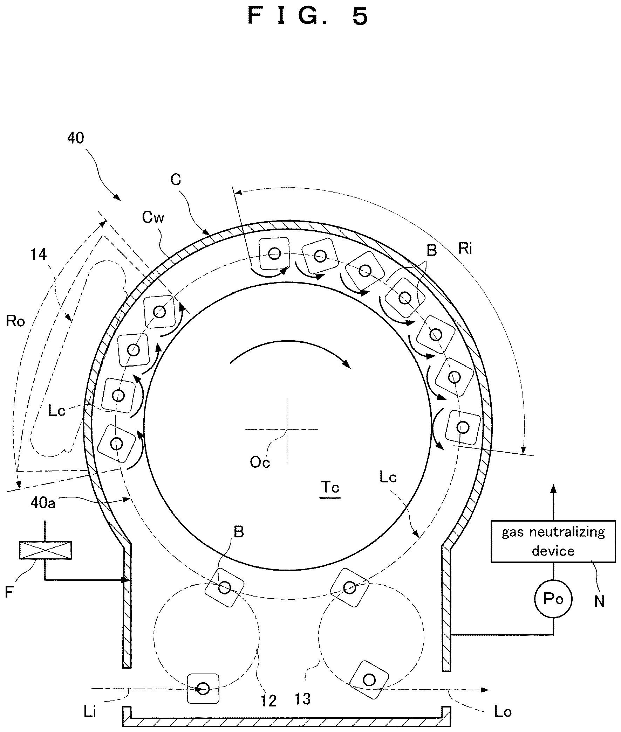

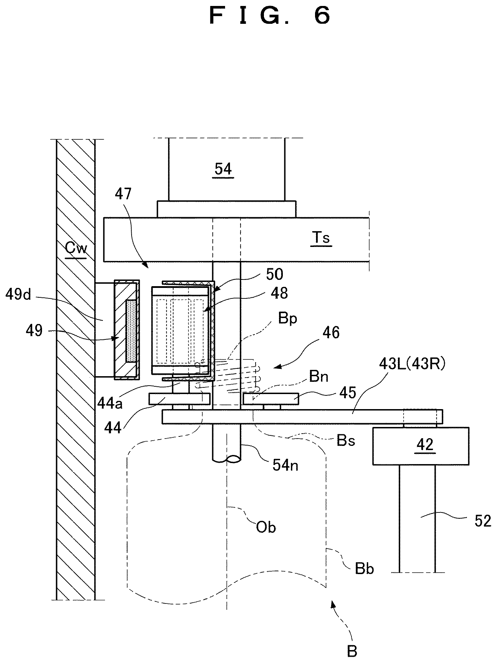

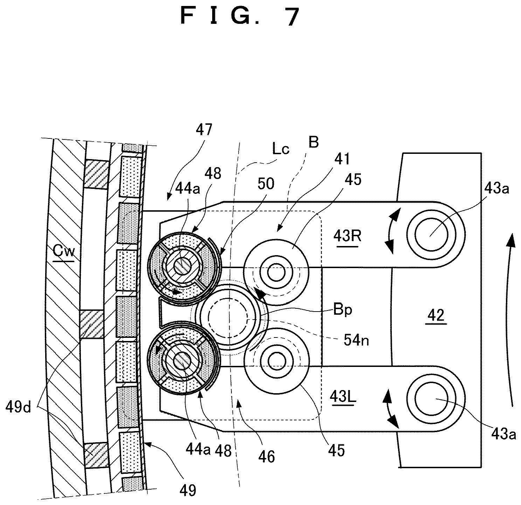

Referring to FIGS. 5 to 8, a second embodiment of an electron beam sterilization apparatus according to the present invention will be described below.

In the electron beam sterilization apparatus, an irradiation object to be sterilized is a container B and a container inner-surface sterilizer 40 is disposed upstream of a filling device (not shown) for filling the container B with contents in axenic conditions. The same configurations as those of the first embodiment are indicated by the same reference numerals and the detailed explanation thereof is omitted.

As shown in FIGS. 5 to 8, in a chamber C for shielding corrosive gas, the container conveying device 40a of the container inner-surface sterilizer 40 includes a rotary table Tc rotatable about a rotation axis Oc, container holding devices 41 that are disposed at regular intervals on the outer periphery of the top of the rotary table Tc and hold the containers B via neck parts Bn, and container lifting devices 51, each raising and lowering the container B via the container holding device 41. Moreover, a circular passage Lc for conveying the containers B is formed on the outer periphery of the rotary table Tc. Reference character Ri denotes an inner-surface irradiation region (electron beam irradiation region) of an electron beam on the circular passage Lc. The container B transferred to the circular passage Lc from a feed passage Li via a feed wheel 12 is lifted by the container lifting device 51 at the entrance of the inner-surface irradiation region Ri. Then, an irradiation nozzle 54n of an electron beam irradiation device 54 is inserted into the container B through a mouth Bp and sterilizes the inner surface of the container B. The container B is lowered at the exit of the inner-surface irradiation region Ri by the container lifting device 51 so as to remove the irradiation nozzle 54n from the mouth Bp. Then, the sterilized container B is discharged to a discharge passage Lo through a discharge wheel 13.

### [Container holding device]

On the outer periphery of the rotary table Tc, lifting bases 42 are disposed at regular intervals so as to rise and lower via lifting rods 52. The container holding device 41 includes a pair of right and left neck holding arms 43R and 43L supported so as to open and close around respective support shafts 43a, 43a on the lifting base 42. The right and left neck holding arms 43R and 43L each include a driving roller 44 rotatably supported about a vertical axis on the distal end and an idling roller 45 rotatably supported about a vertical axis in an intermediate part. In response to the opening and closing of the neck holding arms 43R and 43L, the four rollers 44 and 45 open and hold the neck part Bn of the container B. For an arm opening/closing device for opening and closing the neck holding arms 43R and 43L, known techniques of cams or fluid-driven or electric cylinders are applied, which is not shown. The neck holding arms 43R and 43L are opened and closed at a feed position corresponding to the feed wheel 12 and a discharge position corresponding to the discharge wheel 13, so that the container B is fed to be held and is released to be discharged.

### [Container lifting device]

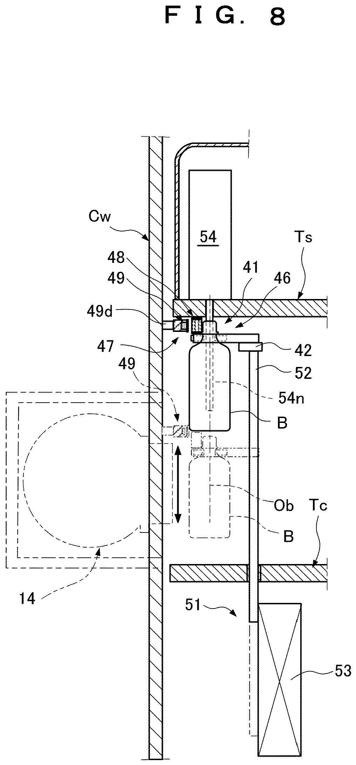

As shown in FIG. 8, in the container holding device 41, the lifting base 42 is supported on the outer periphery of the rotary table Tc so as to be raised and lowered in a predetermined range by the container lifting device 51. Specifically, the container lifting device 51 includes the lifting rod 52 that is suspended from the lifting base 42 and is slidably fit into a guide hole on the outer periphery of the rotary table Tc, and a lifting drive unit 53 that is under the rotary table Tc and raises and lowers the container holding device 41 in a predetermined range via the lifting rod 52. The lifting drive unit 53 is a known drive unit, e.g., a cam mechanism, a fluid-driven cylinder, or an electric cylinder.

Above the rotary table Tc, a support table Ts is installed so as to rotate in synchronization with the rotary table Tc. On the outer periphery of the support table Ts, the nozzle-type electron beam irradiation devices 54 are installed for the respective container holding devices 41 at regular intervals. The electron beam irradiation device 54 includes the irradiation nozzle 54n that extends downward through the support table Ts so as to be inserted into the mouth Bp of the container B. An electron beam is emitted from the irradiation opening at the lower end of the irradiation nozzle 54n. Electron beams emitted from the irradiation nozzle 54n collide with air molecules in the chamber C and extend in a spindle shape, thereby sterilizing the inner surface of the container B.

### [Container rotating device]

It is known that if resin making up the container B is excessively irradiated with an electron beam, the resin may be deteriorated or discolored. The amount of irradiation needs to be correctly kept in a proper range. Thus, in the sterilization of the inner surface of the container B, if a body Bb is particularly rectangular in cross section as illustrated in the drawing or if the body Bb rapidly increases in diameter particularly from the small-diameter neck part Bn to a shoulder part Bs of the body Bb, a distance from the irradiation opening to the inner surface of the irradiation nozzle 54n fluctuates in the circumferential direction. Moreover, the formation of asperities for decoration and gripping on the body Bb leads to irregular distances from the irradiation opening to the inner surface. Thus, it is difficult to keep the irradiation amount of an electron beam in the proper range.

In the second embodiment, the container rotating device (rotating device) 46 rotates the container B about a container axis Ob and an electron beam is evenly emitted to the uneven inner surface of the container B, so that the irradiation amount of an electron beam can be kept in the proper range and the overall inner surface of the container B can be evenly sterilized.

### [Magnetic-type driving force transmission mechanism]

The container rotating device 46 includes a magnetic-type driving force transmission mechanism 47. The magnetic-type driving force transmission mechanism 47 includes a pair of right and left ring magnet assemblies 48, each being attached to the upper part of a vertical interlocking shaft 44a supporting the driving roller 44, and a circular strip magnet assembly 49 circumferentially disposed on a fixed-side member (a partition wall Cw of the chamber C) over the inner-surface irradiation region Ri so as to surround the rotation axis Oc. The ring magnet assembly 48 and the strip magnet assembly 49 are substantially identical in configuration to the ring magnet assembly 33 and the strip magnet assembly 32 of the first embodiment. The ring magnet array and strip magnet array that include alternately arranged N-pole and S-pole magnets are attached to a magnet mounting member. The ring and strip magnet assemblies are respectively covered with a coating member that is made of corrosion-resisting materials having low magnetic permeability and a cover member 49c. The strip magnet assembly 49 is installed on the inner surface of the partition wall Cw of the chamber C via a support member 49d. Reference numeral 50 denotes a magnetic field shield that mainly shields a magnetic field generated from the ring magnet assembly 48 so as to prevent adverse effects on radiation in the irradiation nozzle 54n. The magnetic field shield 50 is made of high magnetic permeability plate materials, i.e., Mu-metal or permalloy and includes an anti-corrosive plate made of a stainless material or a titanium alloy (or an anti-corrosive coating layer covered with a stainless material or a titanium material) .

In this configuration, the container B conveyed on the circular passage Lc while being held by the container holding device 41 is lifted by the lifting drive unit 53 at the entrance of the inner-surface irradiation region Ri. Meanwhile, in the container rotating device 46, the ring magnet assembly 33 of the magnetic-type driving force transmission mechanism 47 approaches the strip magnet assembly 32. When the container B is moved along the circular passage Lc, the ring magnet assembly 33 is rotated by the effects of the magnetic force of the ring magnet assembly 33 and the strip magnet assembly 32. Then, the rotation of the ring magnet assembly 33 rotates the driving roller 44 via the interlocking shaft 44a, allowing the container B to rotate about the container axis Ob. When the container B enters the inner-surface irradiation region Ri, the irradiation nozzle 54n is inserted into the body Bb from the mouth Bp of the container B and sterilizes the inner surface of the container B. When the container B approaches the exit of the inner-surface irradiation region Ri, the irradiation nozzle 54n is removed from the body Bb through the mouth Bp of the container B and the ring magnet assembly 33 is separated from the strip magnet assembly 32 so as to stop the rotation of the container B.

### [Effect of Second Embodiment]

According to the second embodiment, in the magnetic-type driving force transmission mechanism 47, the strip magnet assembly 49 fixed along the circular passage Lc and the moving ring magnet assembly 48 convert a circular motion into a rotational motion, allowing the container B to rotate about the container axis Ob in a noncontact manner. Thus, the magnetic-type driving force transmission mechanism 47 can considerably reduce the occurrence of dust and dirt caused by contact or abrasion, thereby preventing the contamination of the container.

In the second embodiment, the right and left neck holding arms 43R and 43L each include the driving roller 44 at the distal end and the idling roller 45 in the intermediate part. The present invention is not limited to this configuration. The ring magnet assembly 48 and the strip magnet assembly 49 may be installed with the idling rollers 45 at the distal ends of the neck holding arms 43R and 43L and the driving rollers 44 in the intermediate parts of the neck holding arms 43R and 43L as long as the arms do not interfere with ascent and descent.

Alternatively, as indicated by virtual lines, an outer-surface irradiation region Ro of an electron beam may be formed on the circular passage Lc by placing an electron beam irradiation device 14 in a front region of the inner-surface irradiation region Ri, a rear region of the inner-surface irradiation region Ri, or a region at least partially overlapping the inner-surface irradiation region Ri. Needless to say, only the outer-surface irradiation region Ro of an electron beam may be provided.

### [Modifications of the magnetic-type driving force transmission mechanism]

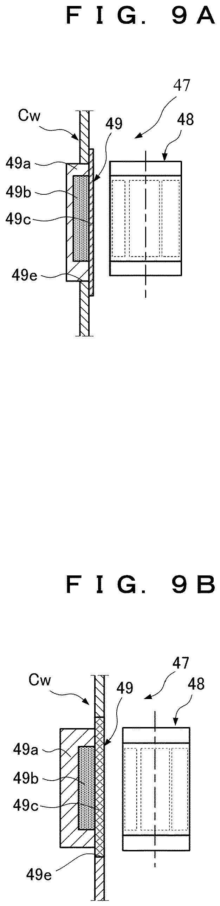

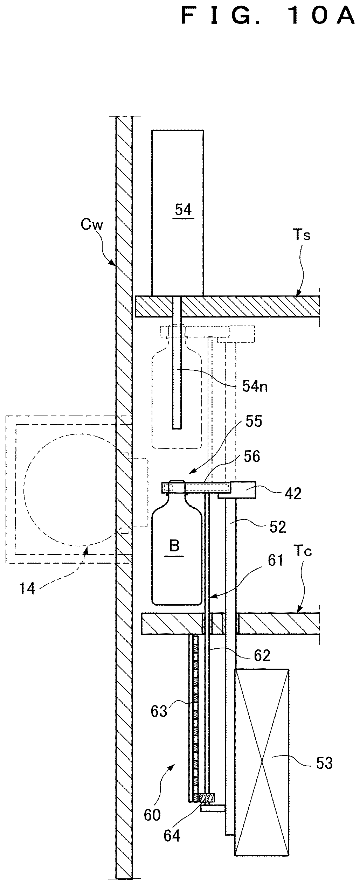

In the second embodiment, the ring magnet assembly 48 and the strip magnet assembly 49 in the magnetic-type driving force transmission mechanism 47 are fully disposed in the chamber C. As shown in FIG. 9A, the strip magnet assembly 49 may be combined with the partition wall Cw of the chamber C (across the inside and outside of the partition wall Cw). Alternatively, as shown in FIG. 9B, the strip magnet assembly 49 may be installed outside the partition wall Cw of the chamber C. Moreover, as shown in FIGS. 10A to 10C, a magnetic-type driving force transmission mechanism 60 may be provided to convert a vertical motion of the lifting base 42 into a rotational motion of the driving roller 44. In the explanation of a first modification (FIG. 9A), a second modification (FIG. 9B), and a third modification (FIGS. 10A to 10C), the same members as those of the second embodiment are indicated by the same reference numerals and the detailed explanation thereof is omitted.

### [First modification of the magnetic-type driving force transmission mechanism]

As shown in FIG. 9A, an opening 49e like a circular band is formed on the partition wall Cw along the circular passage Lc. The strip magnet assembly 49 combined with the partition wall Cw of the chamber C (across the inside and outside of the partition wall Cw) includes a strip magnet array 49b that is mounted in the opening 49e via a magnet mounting member 49a and include alternately arranged S-pole and N-pole magnets, and a corrosion-resisting coating member 49c with low magnetic permeability (or a corrosion-resisting coating layer having low magnetic permeability) that covers the magnet mounting member 49a and the strip magnet array 49b on the inner surface of the partition wall Cw.

### [Second modification of the magnetic-type driving force transmission mechanism]

Alternatively, as shown in FIG. 9B, the strip magnet assembly 49 mounted outside the partition wall Cw of the chamber C has the coating member 49c made of corrosion-resisting materials having low magnetic permeability in the strip-shaped opening 49e formed on the partition wall Cw. The ring magnet array 49b is attached with the magnet mounting member 49a outside the coating member 49c. Since the partition wall Cw has corrosion resistance, the opening 49e and the coating member 49c can be omitted by forming the partition wall Cw made of a material having low magnetic permeability. This can reduce the cost of the apparatus.

According to the first and second modifications, the strip magnet array 49b can be easily separated from an atmosphere of corrosive gas in the chamber C, thereby reducing the occurrence of corrosion. Since the partition wall Cw serves as the support member of the strip magnet assembly 49, the number of members can be reduced. Moreover, a space in the chamber C can be effectively used.

### [Third modification of the magnetic-type driving force transmission mechanism]

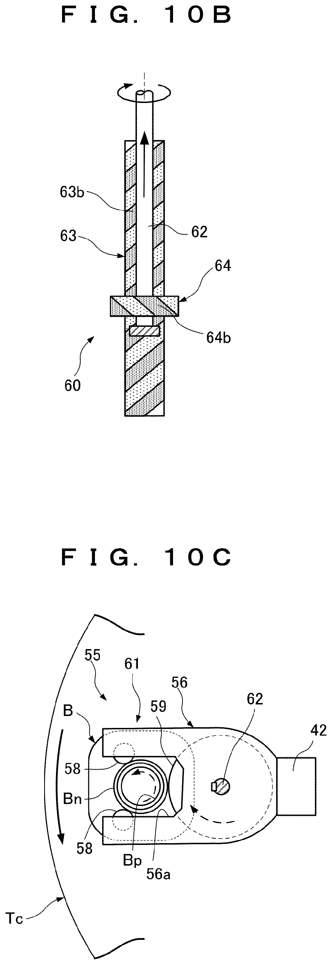

Referring to FIGS. 10A, 10B, and 10C, a third modification will be described below. A container holding device 55 that is raised and lowered in a predetermined range via the lifting rod 52 by the lifting drive unit 53 includes a bifurcated neck holding arm 56 that extends outward from the lifting base 42 and has a recessed part 56a, a pair of right and left idling elastic rollers 58 that are opposed to each other at the entrance of the recessed part 56a of the neck holding arm 56 and retain the neck part Bn, and a driving roller 59 that is disposed behind the recessed part 56a and comes into contact with the neck part Bn.

A container rotating device 61 includes a rotation shaft 62 with the driving roller 59 attached to the upper end of the rotation shaft 62, and the magnetic-type driving force transmission mechanism 60 provided on the lower part of the rotation shaft 62. The rotation shaft 62 is suspended in parallel with the lifting rod 52 and penetrates the rotary table Tc in a slidable and rotatable manner.

The magnetic-type driving force transmission mechanism 60 has the same effects as a helical gear mechanism and includes a strip magnet assembly 63 suspended from the rotary table Tc in parallel with the rotation shaft 62 and a ring magnet assembly 64 fixed at the lower end of the rotation shaft 62. The strip magnet assembly 63 has a linear strip magnet array 63b on the magnet mounting member. In the strip magnet array 63b, S-pole and N-pole magnets alternately arranged at regular intervals in the conveying direction are inclined at a predetermined angle (30° to 60°) with respect to the moving direction (vertical line) of the ring magnet assembly 64, so that the magnets coincide with the teeth of a helical rack. The ring magnet assembly 64 includes a magnet support cylinder fixed to the rotation shaft 62 via a key, and a ring magnet array 64b that includes S-pole and N-pole magnets alternately arranged at regular intervals (angles) on the outer periphery of the magnet support cylinder so as to coincide with the strip magnet array 63b. The S-pole and N-pole magnets of the ring magnet array 64b disposed for the respective magnets of the strip magnet array 63b are inclined so as to coincide with the teeth of a helical gear that is inclined with respect to the moving direction. Thus, the ring magnet assembly 64 is moved along the strip magnet assembly 63 so as to rotate about the rotation shaft 62 by the application of a magnetic force. This converts a vertical motion of the lifting base 42 into a rotational motion of the driving roller 59.

A gap between the rotary table Tc and the partition wall Cw of the chamber C is covered with a noncontact gas shield member, e.g., a labyrinth seal, thereby preventing corrosive gas in the chamber C from entering a space in a noncorrosive atmosphere at the bottom of rotary table Tc. This can prevent corrosion of the magnetic-type driving force transmission mechanism 55, thereby eliminating the need for an anti-corrosive cover member, a coating member, or a coating layer.

In the configuration, when the container B enters the inner-surface irradiation region Ri, the lifting rod 52 is lifted with the lifting base 42 by the lifting drive unit 53 in the magnetic-type driving force transmission mechanism 60 so as to lift the container B; meanwhile, the ring magnet assembly 64 moves along the strip magnet array 63b, thereby applying a magnetic force so as to rotate the ring magnet assembly 64. Thus, the irradiation nozzle 54n is inserted into the body Bb from the mouth Bp and the container B is rotated about the container axis Ob via the rotation shaft 62 and the driving roller 59. Hence, an electron beam is sequentially emitted from the irradiation opening of the irradiation nozzle 54n into the mouth Bp of the container B, the neck part Bn, the shoulder part Bs, and the body Bb, achieving effective sterilization according to the irradiation time and the irradiation distance.

According to the third modification of the magnetic-type driving force transmission mechanism, a rotary force can be generated in a noncontact manner by the magnetic-type driving force transmission mechanism 60 including the fixed strip magnet array 63b and the ring magnet assembly 64 that linearly moves along the strip magnet array 63b. Thus, the magnetic-type driving force transmission mechanism 60 can considerably reduce the occurrence of dust or dirt caused by contact or abrasion, thereby preventing contamination of the container B.

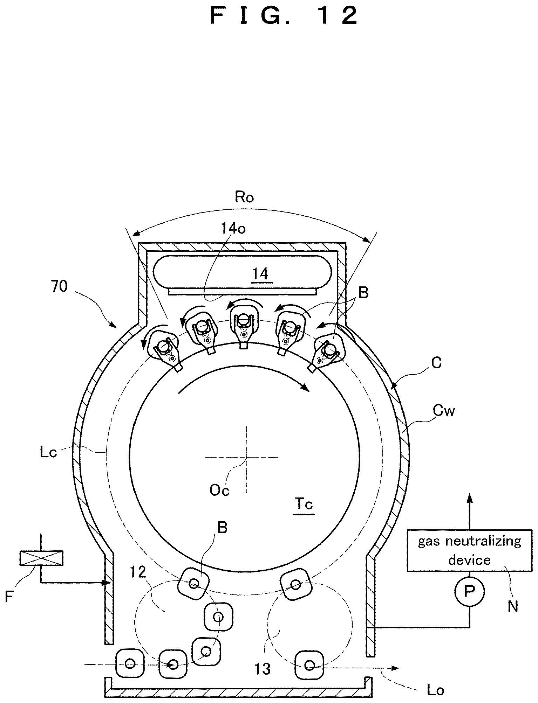

In the second embodiment, the inner surface of the rotated container B is sterilized by the container inner-surface sterilizer 40. The electron beam irradiation device 14 may be disposed outside or inside the circular passage Lc indicated by the virtual line in FIG. 12, and the outer surface of the rotated container B may be sterilized by the container outer-surface sterilizer 11 in the outer-surface irradiation region Ro. Alternatively, the outer surface and the inner surface of the container B may be sequentially sterilized by a container inner-outer-surface sterilizer on the single circular passage Lc in the outer-surface and inner-surface irradiation regions Ro and Ri. The rotation of the container B may be stopped in the inner-surface irradiation region Ri.

In the third modification, as indicated by a virtual line in FIG. 10A, the electron beam irradiation device 14 is disposed in the outer-surface irradiation region where the container B is raised or lowered by the lifting drive unit 53. This can sterilize the overall outer surface of the rotated container B.

### Third Embodiment

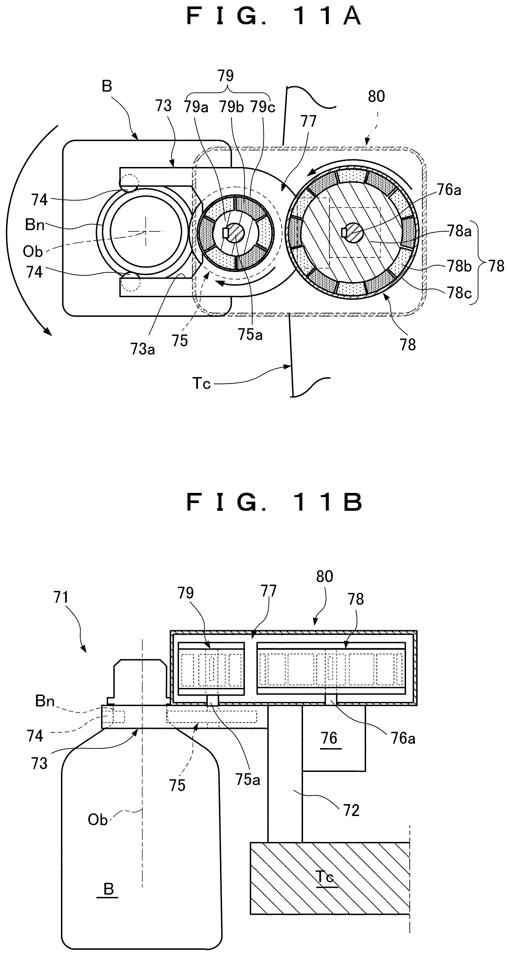

Referring to FIGS. 11A, 11B, and 12, another embodiment of a magnetic-type driving force transmission mechanism will be described below.

An electron beam sterilization apparatus discussed below relates to a container outer-surface sterilizer in which an irradiation object to be sterilized is a container B. The same members as those of the foregoing embodiments are indicated by the same reference numerals and the detailed explanation thereof is omitted.

As shown in FIG. 12, a container outer-surface sterilizer 70 includes a rotary table Tc that is rotatable about a rotation axis Oc in a chamber C for shielding corrosive gas. On the outer periphery of the rotary table Tc, container holding devices 71, each of which holds the container B via a neck part Bn, are installed at regular intervals. The container holding devices 71 convey the containers B along a circular conveying passage Lc. Moreover, an electron beam irradiation device 14 is installed on the outer periphery of the circular conveying passage Lc. The outer surface of the container B is irradiated with an electron beam from the electron beam irradiation device 14 in an inner-surface irradiation region Ri (FIG. 5) corresponding to an irradiation opening 14o.

As shown in FIG. 11A, the container holding device 71 includes a bifurcated clamp body 73 on the upper end of a support 72 raised on the rotary table Tc. The clamp body 73 has a recessed part 73a where the neck part Bn is inserted and removed. A pair of right and left idling elastic rollers 74 opposed to each other is disposed at the entrance of the recessed part 73a such that each of the idling elastic rollers 74 is rotatable about an axis parallel to a container axis Ob. Behind the recessed part 73a, a driving roller 75 is supported by a rotation shaft 75a parallel to the container axis Ob. Then, the neck part Bn is transferred into the recessed part 73a so as to be held by the two elastic rollers 74 and the driving roller 75 via the neck part Bn.

A magnetic-type driving force transmission mechanism 77 is provided between an output shaft 76a of a driving motor 76 attached to the support 72 and the rotation shaft 75a of the driving roller 75. The magnetic-type driving force transmission mechanism 77 transmits a rotational motion of the output shaft 76a of the driving motor 76 to a rotational motion of the rotation shaft 75a of the driving roller 75, which is the same effect as a gear mechanism. A driving-side ring magnet assembly 78 attached to the output shaft 76a of the driving motor 76 includes a ring magnet array 78b that includes S-pole and N-pole magnets alternately arranged at regular intervals (angles) on the outer periphery of a magnet mounting member 78a, and a driving-side coating member 78c that is a coating covering the ring magnet array 78b. A receiving-movement-side ring magnet assembly 79 that is brought close to the driving-side ring magnet assembly 78 and is attached to the rotation shaft 75a of the driving roller 75 includes a ring magnet array 79b that includes S-pole and N-pole magnets alternately arranged at regular intervals (angles) on the outer periphery of a magnet mounting member 79a, and a receiving-movement-side coating member 79c that is a low-magnetic permeability and anti-corrosive coating covering the ring magnet array 79b.

Reference numeral 80 denotes a magnetic field shield that is made of high magnetic permeability plate materials, i.e., Mu-metal or permalloy and includes an anti-corrosive plate made of a stainless material or a titanium alloy (or an anti-corrosive coating layer covered with a stainless material or a titanium material). The magnetic field shield 80 can shield a magnetic field that is generated from the magnetic-type driving force transmission mechanism 77 and reaches an electron beam, and shield corrosive gas that reaches the magnetic-type driving force transmission mechanism 77 from the inside of the chamber C.

In the third embodiment, the magnetic-type driving force transmission mechanism 77 that transmits power in a noncontact manner is provided between the driving-side ring magnet assembly 78 to rotate and the receiving-movement-side ring magnet assembly 79 to be rotated, thereby preventing the occurrence of dust or dirt caused by contact or abrasion during power transmission. Thus, the container B is not contaminated by dust or dirt.

In the first embodiment, regarding the conveying passage L for conveying the preform P held by the container holding part 25, the linear first half part La was discussed as a passage for sterilizing the rotated preform P. The passage for sterilization is not limited and may be the circular passage Lc described in the second embodiment or the third embodiment.

The first to third embodiments are merely exemplary and are not restrictive in all the aspects. The scope of the present invention is not indicated by the foregoing description but the claims. The scope of the present invention is intended to include meanings equivalent to the claims and all changes in the scope. Among the configurations described in the first to third embodiments, the configurations other than those described in the claims are optional and thus can be deleted and changed as appropriate.

## Claims

1. An electron beam sterilization apparatus for sterilization with an electron beam emitted from an electron beam irradiation device to an irradiation object,
the sterilization apparatus comprising:
a conveying device that conveys a held irradiation object;
a rotating device that is provided on the conveying device and rotates the irradiation object; and
a magnetic-type driving force transmission mechanism that transmits a driving force in a non-contact manner between a driving member and a receiving movement member of the rotating device,
the driving force transmission mechanism including a magnet array provided for each of the driving member and the receiving movement member that are moved while being opposed to each other with a predetermined gap, the magnet array including magnets alternately arranged with different magnetic poles.

2. The electron beam sterilization apparatus according to claim 1, further comprising an interference preventing member between the driving force transmission mechanism and the irradiation object, the interference preventing member preventing a magnetic force generated from the magnetic-type driving force transmission mechanism from adversely affecting the electron beam.

3. The electron beam sterilization apparatus according to claim 1 or 2, wherein the magnet array is covered with a coating member made of a corrosion-resisting material having low magnetic permeability.

4. The electron beam sterilization apparatus according to claim 1 or 2, wherein the driving member is a strip magnet assembly disposed on a fixed-side member along a conveying direction of the irradiation object, and the receiving movement member is a ring magnet assembly provided on a movable-side member of the conveying device.

5. The electron beam sterilization apparatus according to claim 1 or 2, further comprising a chamber surrounding the electron beam irradiation device and the irradiation object with a partition wall that is capable of shielding an X-ray with low magnetic permeability and is made of a corrosion-resisting material,
the magnet array of the receiving movement member is disposed in the chamber, and
the magnet array of the driving member is disposed outside the chamber via a coating member made of a corrosion-resisting material having low magnetic permeability.
